# EUROPEAN PATENT SPECIFICATION

(11) **EP 3 865 143 B1**
(45) Date of publication and mention of the grant of the patent: **24.12.2025**
(21) Application number: 20020074.9
(22) Date of filing: 17.02.2020
(51) Int. Cl.: A61K 38/00, C07K 14/47, C12P 21/00, G01N 30/02

(54) **PREPARATION OF FETUIN A**
HERSTELLUNG VON FETUIN A
PRÉPARATION DE FETUINE A

(43) Date of publication of application: 18.08.2021
(73) Proprietor: PreviPharma GmbH, 68167 Mannheim (DE)
(72) Inventor: Kießig, Stephan T., 69168 Wiesloch (DE); Berger, Tina, 64625 Bensheim (DE); Mandago, Maurice, 69250 Schönau (DE); Gruber, Ricarda, 69256 Mauer (DE); Gerding, Hanne, 68229 Mannheim (DE); Müller, Dirk, 68165 Mannheim (DE)
(74) Representative: Sacht-Gorny, Gudrun

(56) References cited:
- WO-A2-02/094201
- US-A- 4 816 437
- CHAILURKIT L ET AL: "The relationship of fetuin-A and lactoferrin with bone mass in elderly women", OSTEOPOROSIS INTERNATIONAL ; WITH OTHER METABOLIC BONE DISEASES, SPRINGER-VERLAG, LO, vol. 22, no. 7, 21 October 2010 (2010-10-21), pages 2159 - 2164, XP019911457, ISSN: 1433-2965, DOI: 10.1007/S00198-010-1439-3
- LEVIN Y ET AL: "Multidimensional protein fractionation of blood proteins coupled to data-independent nanoLC-MS/MS analysis", JOURNAL OF PROTEOMICS, ELSEVIER, AMSTERDAM, NL, vol. 73, no. 3, 3 January 2010 (2010-01-03), pages 689 - 695, XP026814974, ISSN: 1874-3919, [retrieved on 20091105]
- ANONYMOUS: "Alpha 2 HS Glycoprotein, Human Plasma | Athens Research & Technology", 20 August 2016 (2016-08-20), XP093057150, Retrieved from the Internet <URL:https://web.archive.org/web/20160820164050/https://www.athensresearch.com/products/human-proteins/alpha-2-hs-glycoprotein-human-plasma> [retrieved on 20230623]

## Description

The present invention relates to a method for preparing Fetuin A (alpha-2-HS glycoprotein) from a biological liquid. Moreover, the present invention relates to a pharmaceutical composition comprising a protein preparation obtainable by the above method for use in the treatment of fetuin A deficiency.

Fetuin A is also known as alpha-2-HS glycoprotein and belongs to the Fetuin class of plasma-binding proteins which manage transport and availability of various substances in the bloodstream. There are further synonyms for Fetuin A respectively alpha-2-HS glycoprotein, namely alpha-2-HS, A2HS, AHS and HSGA. Fetuins occur in large quantities in fetal blood. Besides serum albumin there are two further types of Fetuins, namely Fetuin A and Fetuin B. Normal serum concentrations of Fetuin A in healthy adults range between 0.3 and 1.0 g/L.

Fetuin A is mainly expressed from the liver, but also from the kidney and choroid plexus. Fetuin A is highly expressed in the non-collagenous bone matrix during fetal life and affects bone growth via antagonizing the effects of transforming growth factor beta (Schlieper G., Westenfeld R., Brandenburg V., Ketteler M., Inhibitors of calcification in blood and urine. Semin Dial 2007, 20: 113-121; Heiss A., DuChesne A, Denecke B., Grotzinger J., Yamamoto K., Renne T., Jahnen-Dechent W., Structural basis of calcification inhibition by alpha 2-HS glycoprotein/fetuin-A. Formation of colloidal calciprotein particles. J Biol Chem 2003, 278: 13333-13341; Quelch K.J., Cole W.G., Melick R.A., Non-collagenous proteins in normal and pathological human bone. Calcif Tissue Int 1984; 36: 545-549). Lack of Fetuin A has been shown to result in severe systemic calcification in mice and humans (Ketteler M., Bongartz P., Westenfeld R., Wildberger J., Mahnken A., Bohm R., Metzger T., Wanner C., Jahnen-Dechent W., Floege J., Association of low fetuin-A (AHSG) concentrations in serum with cardiovascular mortality in patients on dialysis: a cross-sectional study. Lancet 2003; 361: 827-833; Reynolds J.L., Skepper J.N., McNair R., Kasama T., Gupta K., Weissberg P.L., Jahnen-Dechent W., Shanahan C.M., Multifunctional roles for serum protein fetuin-a in inhibition of human vascular smooth muscle cell calcification. J Am Soc Nephrol 2005, 16: 2920-2930). Fetuin A facilitates the removal of hydroxyapatite crystals by increasing the calciprotein particles in the bloodstream and, therefore, inhibiting intravascular calcification (Heiss A. et al., see above).

The bone is a living, active tissue constantly remodeling itself through bone resorption and formation. An imbalance between the two stages leads to osteoporosis. Osteoporotic bones are spongier (porous) and have lower density or mass and contain abnormal tissue structure. Consequently, when bones become weak and an increased risk of spontaneous fractures (hip, spine or wrist) occurs. The basic framework of bones is given by collagen fibers. The minerals (calcium, phosphate) are responsible for hardening the bones. In both, men and women, osteoporosis onset with advanced age (in women 5 - 7 years after menopause). The first symptoms are permanent pain, losing height, stooped or hunched posture, limiting mobility often leading to isolation or depression. The current standard of care is based on a basic therapy using calcium, phosphate and vitamin D in combination with other drugs. Also the bone resorption can be targeted by slowing medications (e.g. bisphosphonate, estrogen, or specific monoclonal antibodies acting on osteoclasts). Sometimes bone building promoting drugs (e.g. fluorides, or parathyroid hormones) may increase the activity of bone-building cells (osteoblasts). Nevertheless, the current therapy is not always sufficient to prevent vertebral and bone fractures. One main limitation in the currently used drugs is their lower efficacy on non-vertebral fracture reduction than on vertebral fracture. Moreover, there are severe side effects and potential adverse events on long-term use. Additionally, the treatment of osteoporosis and of related bone fractures is very cost intensive.

In general, people with age of ≥ 50 years have a high risk of osteoporotic bone fractures. In 2010 158 million people worldwide suffered from osteoporosis, this number will be almost doubled up to 2040 to 310 million individuals. Nearly half of all women are expected to suffer an osteoporotic fracture in their lifetime (Sweet MG, Sweet JM, Jeremiah MP, Galazka SS. Diagnosis and treatment of osteoporosis. Am Fam Physician 2009; 79: 193-200). There are 7 - 20 million people expected in 2050 only with osteoporosis-related hip fractures. Twenty percent of the patients with hip fracture become functionally dependent and require long-term nursing care (Jaglal SB, Sherry PG, Schatzker J. The impact of hip fracture in Ontario and its consequences. Can J Surg 1996; 2: 105-111).

The exact mechanism of Fetuin A in bone mineralization has not been completely elucidated. In the course of an *in vitro* rat study, Toroian et al. noted that Fetuin A stimulated calcification within bone (Toroian D., Lim J.E., Price P.A., The size exclusion characteristics of type I collagen: implications for the role of non-collagenous bone constituents in mineralization. J Biol Chem 2007, 282(31): 22437-22447). There are limited human studies on Fetuin A to define its exact mechanism (lx J.H., Wassel C.L., Bauer D.C., Toroian D., Tylavsky F.A., Cauley J.A., Harris T.B., Price P.A., Cummings S.R., Shlipak M.G., Fetuin-A and bone mineral density in older persons: The Health Aging and Body Composition (Health ABC) study. J Bone Miner Res 2009, 24: 514-521; Chailurkit L., Kruavit A., Rajatanavin R., Ongphiphadhanakul B., The relationship of fetuin-A and lactoferrin with bone mass in elderly women. Osteoporos Int 2011; 22: 2159-2164). WO 02/094201 discloses a method of purifying bovine fetuin A to obtain a preparation with 99% purity, and the use of this preparation in medicine.

US 4 816 437 A discloses methods and procedures of purifying fetuin A, including human fetuin A.

Fetuin A is commercially available, especially prepared from fetal calf serum. It is known to prepare Fetuin A by ammonium sulfate precipitation or by cold ethanol precipitation according to Spiro (Spiro R. G., Journal of Biological Chemistry 235, 10: 2860, 1960). Moreover, a one-step affinity purification of Fetuin from fetal bovine serum was developed on the basis of wheat germ agglutinin affinity separation (Cartellieri S. et al., Biotechnol. Appl. Biochem. 2002, 35 (2): 83 - 9). The preparation from fetal serum is widely preferred because the content of Fetuin A in plasma is much higher than in adult plasma.

There is still a need for effective preparation methods for Fetuin A from biological liquids, especially from human plasma, preferably from adult plasma. The present invention has the object to provide an efficient preparation method for Fetuin A from biological liquids and especially from plasma or a plasma-derived preparation or from other sources. This object is solved by a method as depicted in claim 1. Preferred embodiments are outlined in the dependent claims. Furthermore, the object is solved by a pharmaceutical composition as depicted in the further independent and dependent claims.

The inventive method for preparing Fetuin A (alpha-2-HS glycoprotein) from a biological liquid comprises a chromatography step of adsorption of Fetuin A on a chelate chromatography material, wherein the material is charged with a doubly charged cation. Further, the chromatography step comprises eluting the Fetuin A from the material. Surprisingly, the inventors have found that Fetuin A can be very efficiently purified from biological liquids by using chelate-forming carriers for chromatography. The chelate chromatography material used for the inventive method is an immobilized metal affinity chromatography (IMAC) resin. In general, by this method the proteins or peptides are separated according to the affinity for metal ions that have been immobilized by chelation to an insoluble matrix. At pH values around neutral, the amino acids histidine, tryptophan, and cysteine form complexes with the chelated metal ions like Zn²⁺, Cu²⁺, Cd²⁺, Hg²⁺, Co²⁺, Ni²⁺, and Fe²⁺. The adsorbed proteins or peptides can then be eluted by reducing the pH, or by increasing the mobile phase ionic strength, or by adding ethylene-diamine tetraacidic acid (EDTA) to the mobile phase. Conventionally, this technique is especially useful for purifying recombinant proteins which are expressed as poly-histidine fusions and for purifying membrane proteins and protein aggregates, wherein detergents or high ionic strength buffers are generally required. As found by the inventors, such a chelate chromatography resin is unexpectedly useful for preparing Fetuin A from biological fluids.

In preferred embodiments the chelate chromatography resin comprises immobilized iminodiacetic acid (IDA) and/or nitrilotriacetic acid (NTA) as a ligand. Both types of chelate chromatography resins have been applied very successfully in preparing Fetuin A from a biological liquid.

The chromatography step of the inventive method may be performed as column chromatography or batch chromatography. Especially for industrial purposes column chromatography may be preferred.

In preferred embodiments the biological liquid is plasma or a plasma-derived preparation, especially human plasma or a human plasma-derived preparation. Nevertheless, the inventive method may be used to prepare Fetuin A from other sources, e.g. from bovine or other plasma. Other sources may be other body fluids containing Fetuin A or, e. g., cell culture supernatants from cells expressing Fetuin A naturally or based on recombinant techniques.

It is especially preferred to integrate the inventive method into usually practiced fractionation processes of human plasma in order to use the limited raw material plasma for the preparation of different plasma proteins including the preparation of Fetuin A according to the inventive method. The inventors were able to show that Fetuin A can be purified in a very efficient way from a Cohn fraction, especially from Cohn fraction IV with or without removal of albumin. The Cohn fractionation process is a widely established plasma fractionation process which has been developed in 1946, wherein the Cohn process involves a series of precipitation steps using different ethanol concentrations and varying temperatures and pH values. The process is based on the differential solubility of the diverse plasma proteins under the different conditions. Cohn fraction IV is achieved by 40% ethanol at pH 5.8 at low temperature. The dissolved precipitate (paste) of this step may be preferably used as source material for the inventive method. In an even more preferred embodiment Cohn fraction IV may be used after removal of albumin.

Especially before loading the chromatography material with the biological liquid, the chromatography material has to be loaded (charged) with a doubly charged cation. The doubly charged cation Ca²⁺ is used. For charging the chelate chromatography material with the doubly charged cation a buffer with 10 - 100 mM CaCl₂ or a salt of another doubly charged cation may be used. Especially preferred is a molarity of 50 mM. The doubly charged cation will form a chelate complex with the chromatography material, thereby allowing the specific adsorption of Fetuin A on the chromatography material. The adsorbed Fetuin A can then be eluted with a high degree of purity.

Preferably, the chromatography step is performed at a pH in the range between 4 - 8, preferably between pH 4 - 6, more preferably at pH 5. In other embodiments pH 8 may be prefered. Preferably, all buffers used for the chromatography step have the same pH, e.g. pH 5 or pH 8, especially the buffers used for equilibration, charging, loading, washing and elution. It has been shown by the inventors that especially under such conditions, especially at pH 5, a purification of Fetuin A with very good results can be achieved. Also when using pH 8 good results have been achieved. Preferably, during the whole chromatography step including loading, optionally washing and the following elution the pH value is not essentially changed.

For the chromatography step different kind of buffers may be used, especially buffers which have a good buffering capacity in the acidic range. Especially preferred is acetate buffer. Nevertheless, also other buffers may be used like Tris, HEPES, barbiturate and phosphate buffers. In general, the molarity of the buffer may be used in a range of about 10 - 150 mM. For example, very good results have been achieved by the use of 100 mM acetate buffer.

After charging the chromatographic material with the doubly charged cation the following buffers, especially for loading, optionally washing and elution, preferably do not contain the doubly charged cation of the charging step or any other doubly charged cation. In an especially preferred embodiment the chromatography step comprises a washing step after loading, wherein washing is performed using a buffer not comprising any doubly charged cations. It is especially preferred to apply such a washing step after loading in order to remove all unbound doubly charged cation from the chromatography material, because any doubly charged cation may interfere with the specific chelating interaction between Fetuin A and the doubly charged cation which is immobilized on the chromatography material by chelation and/or which may compete with the ligands at the chromatography material.

Preferably, the buffer as used for elution comprises a cation competing with the cationic group of charged chromatography material. Thus, the elution of the adsorbed Fetuin A is performed by increased ionic strength. Preferably, Na⁺ or other monovalent cations like K⁺ or Li⁺ may be used, preferably in form of the corresponding salts. In an especially preferred embodiment elution is done by an increased Na⁺ concentration, especially by an increased or increasing NaCl concentration. In especially preferred embodiments the elution is performed with a salt gradient, especially in combination with column chromatography. An especially preferred example for such a solution is an increasing NaCl gradient, for example a gradient from 10 mM up to 1.4 M NaCl. The inventors were able to achieve very good results with such an NaCl gradient. Nevertheless, it is also possible to run a stepwise elution with an increased salt concentration. In especially preferred embodiments Fetuin A may be eluted in the range of 150 mM - 500 mM NaCl with a high purity. When analyzing the elution fractions Fetuin A appears as a strong band at about 60 kDa and a weaker band at about 50 kDa on SDS-PAGE as verified by Western Blotting.

It may be preferred to perform the chromatography step in the presence of at least one stabilizer for prevention of degradation of Fetuin A. In preferred embodiments the at least one stabilizer is a doubly charged cation. Additionally or alternatively, albumin or another proteinaceous stabilizer may be used.

To summarize the inventive method for preparation of Fetuin A may comprise the following steps:
(i) providing blood plasma or a plasma fraction comprising Fetuin A, preferably Cohn fraction IV after removal of albumin,
(ii) charging a chelate chromatography material, especially an immobilized metal affinity chromatography material (IMAC), with a doubly charged cation, e. g. with CaCl₂,
(iii) contacting the blood plasma or the plasma fraction with the IMAC material, e.g. by loading a column filled with the chromatography material,
(iv) washing the IMAC material obtained from step (iii) with a first buffer (washing buffer) not comprising any doubly charged positive ions,
(v) eluting the Fetuin A from the washed IMAC material with a second buffer (elution buffer) comprising cations competing with the IMAC resin, wherein preferably the cations may be applied by a salt gradient, preferably a NaCl gradient using a maximal concentration of e.g. 1.4 mol/L NaCl.

During the chromatography optionally Fetuin A may be stabilized by adding one or more stabilizers that prevent the protein from degradation. The stabilizers may be added at the elution step or later. The one or more stabilizers which may be optionally added are preferably doubly charged cations and/or albumin, wherein combinations of doubly charged cations and albumin are preferred.

The pH value in the solution obtained from the elution step may be adjusted to a desired range or value.

In preferred embodiments the purification may be combined with one or more antiviral treatments. Especially the solution obtained from the elution step may be subjected to one or more antiviral treatments as they are known to an expert in the art. Examples of antiviral treatments are solvent/detergent treatments, UV irradiation, nanofiltration, heat treatment, especially dry heat treatment or others. E.g. the optional antiviral treatment may be performed by adding one or more detergents, preferably one or more detergents selected from the group consisting of Tween-20, Tween-80 and Triton-X-100. Additionally or alternatively, one or more other antiviral agents such as a phosphate ester, in particular tri-n-butyl-phosphate (TNBP) may be added. Moreover, filtration steps may be applied like ultrafiltration and, especially preferred, nanofiltration. In especially preferred embodiments combinations of two or more of the aforementioned antiviral treatments may be applied.

Moreover, the obtained product may be provided in a solid, especially dried form, wherein the method may comprise drying or freeze-drying of the eluted Fetuin A directly obtained from elution or obtained from further optional steps after elution. The inventors were able to show that the described method enables to obtain comparably high yields of rather pure Fetuin A from different plasma fractions, especially from plasma fractions which may be obtained from widely used plasma fractionation processes. In comparison to prior art preparation processes for Fetuin A, the method of the present invention enables to obtain increased protein yields and to minimize degradation or denaturation of Fetuin A with a high overall protein purity. Typically, the inventive method allows a purity of more than 60% (w/w) in relation to the total polypeptide mass of the obtained preparation, preferably the purity is 80% (w/w) or even greater.

It is an especially advantageous feature of the inventive method that the method may be integrated into existing plasma fractionation processes. The source material of the inventive method, namely the loading material, may be plasma or plasma intermediates, e.g. cryoprecipitate or cryo-poor plasma or a fraction or waste fraction of a precipitation step in plasma fractionation like the Cohn process or the Kistler-Nitschmann process (a variant of the Cohn process), as long, of course, the material contains Fetuin A. Cryo-poor plasma is typically obtained from the supernatant of blood plasma subjected to being frozen and subsequently thawn. In especially preferred embodiments the waste fraction(s) in the Cohn or Kistler-Nitschmann process of paste I + II + III or waste fraction(s) of paste I + III of the Cohn or Kistler-Nitschmann process or a combination of two or all three of these fractions may be used. Moreover, paste I + II + III or paste I + III of the Cohn or Kistler-Nitschmann process or any fraction or waste fraction thereof containing Fetuin A may be used. In especially preferred embodiments paste IV of the Cohn or Kistler-Nitschmann process or any subfraction, fraction or waste fraction thereof containing Fetuin A may be used as a source material for loading the chromatography material according to the inventive method.

The flow-through of the chromatography step of the inventive method may flow directly into further fractionation steps, like further steps of the Cohn process, for example.

Also disclosed is a protein preparation comprising Fetuin A obtainable or obtained by the described process. Preferably, the preparation comprises at least 60% (w/w), more preferably at least 80% (w/w) alpha-2-HS glycoprotein in relation to total polypeptide mass of the preparation. Albumin and/or Ceruloplasmin and/or transferrin and/or ferritin and/or IgG and/or IgM and/or IgA and/or haptoglobin, for example, or others may be comprised in the composition as further compounds.

Also disclosed is a pharmaceutical composition comprising a protein preparation as described above and at least one pharmaceutically acceptable carrier and/or excipient. The protein preparation of this pharmaceutical composition is obtainable by the inventive method as described above and may be further characterized by an advantageously high purity of Fetuin A, especially a purity of at least 60% (w/w), preferably at least 80% (w/w), in relation to the whole polypeptide or protein content of the composition. The pharmaceutically acceptable excipient or carrier may be a suitable excipient or carrier like, e.g., buffers, stabilizing agents, bulking agents, a preservative or the like as it is known to an expert in the art. The pharmaceutical composition may be a liquid (solution or gel or the like) or in dried form, e. g., lyophilized. If the pharmaceutical composition is in lyophilized form, the composition may be intended for reconstitution with a suitable liquid, e. g. with water or an appropriate buffer. The pharmaceutical composition may be intended for different administration routes, e. g. for oral application in the form of tablets, pills, powders, or the like, or for intravenous or subcutaneous application or intraperitoneal or intramuscular administration routes.

Moreover, the invention comprises a pharmaceutical composition comprising a protein preparation comprising Fetuin A and at least one pharmaceutically acceptable carrier for use in treatment or prophylaxis in patients suffering from Fetuin A deficiency. Generally, the Fetuin A deficiency is independent from the cause of the deficiency, namely inborn or acquired Fetuin A deficiency. According to this aspect of the invention patients suffering from Fetuin A deficiencies may be treated or prophylactically treated with the pharmaceutical composition comprising Fetuin A. Thus, Fetuin A respectively alpha-2-HS glycoprotein according to this invention may be used also to treat patients suffering from acquired or inborn Fetuin A deficiencies in general. Those patients may therefore be at risk to develop an osteoporosis with a high risk for bone fractures.

The protein preparation comprising Fetuin A as component of the pharmaceutical composition is obtainable by the above-defined preparation method of Fetuin A.

The Fetuin A is a human plasma protein, prepared from human plasma or a human plasma-derived preparation.

In further disclosed embodiments not part of the invention, it may be especially preferred to prepare a recombinant Fetuin A, which may be produced and preferably secreted by recombinant cells in cell culture. Thus, it may be especially preferred to prepare Fetuin A according to the inventive method by the use of corresponding cell culture supernatants obtained from recombinant cells which produce Fetuin A and which are prepared by genetic engineering techniques. The cells may be prokaryotic cells like, e.g., *Escherichia coli* or other commonly used cells in recombinant techniques, or insect cells, or, especially preferred, eukaryotic cells, like Chinese hamster ovarian cells (CHO) or human cell lines like HEK (Human Embryonic Kidney) cells, which may be used for recombinant protein production.

The pharmaceutical composition of the present invention is preferably used for those patients who are characterized by a decreased level of Fetuin A, which is preferably lower than 90% confidence interval of healthy subjects (e.g. 90% confidence interval: 0.0094 ... 0.2716 g/L in the chosen control population as shown in Example 4) in comparison. Additionally or alternatively, the level of Fetuin A in the blood of the patient is at least 1% (mol/mol) lower in comparison to the average level found throughout a comparable population of the same species.

In especially preferred embodiments of the described pharmaceutical compositions the pharmaceutical composition is for use in treatment or prophylaxis in patients suffering from osteoporosis and/or in patients on risk of bone fractures and/or in patients on risk of hypercalcification and/or in patients on risk of endochondral extraosseous or heterotopic ossification. Thus, the invention comprises a method for treating a patient suffering from or being at risk of developing a disorder of the bone metabolism in terms of osteoporosis and calcinosis.

Surprisingly, it has been found by the inventors that Fetuin A can be used for the detail in the experimental section Fetuin A deficiency was shown in a diagnostic study. The Fetuin A levels of patients with a proven osteoporosis was compared with the Fetuin A levels of healthy but elderly plasma donors. It has been shown by the inventors that the level of Fetuin A was decreased by the factor 5 (comparing medians) in the patients with osteoporosis. Not all, but the majority of the patients showed the low level of Fetuin A, strongly suggesting that the substitution will improve the bone metabolism in a way, that the major risk for fractures, namely the bone density, can be improved.

As a further aspect, surprisingly, the pharmaceutical composition of the invention may be beneficially applied in dialysis patients on risk for hypercalcification (calciphylaxis). Calciphylaxis is a vascular calcification disorder which may occur in patients with end-stage renal diseases as reviewed by Nigwekar SU: Calciphylaxis, Curr Opin Nephrol Hypertens. 2017 July; 26(4): 276-281 (doi: 10.1097/MNH.0000000000000328). The risk of hypercalcification may be caused by an acquired plasminogen deficiency. Also other reasons can cause the hypercalcification, e.g. chronic inflammation of joints or muscles. The excess of calcium in these patients can be removed by the administration of Fetuin A. Without being bound to any theory, Fetuin A, respectively alpha-2-HS glycoprotein, will bind the access of calcium ions, pack them into calciprotein particles (CPPs) and will transport the calcium to the bones. Therefore, the pharmaceutical compositions of the invention may be also used for treatment and prophylaxis in patients on risk for hypercalcification (calciphylaxis), and, moreover, in patients on risk of endochondral extraosseous or heterotopic ossification.

Moreover, the results as obtained by the inventors allow the conclusion, that Fetuin A, respectively alpha-2-HS glycoprotein, is an independent parameter for the diagnosis of osteoporosis, wherein the inventors analyzed diverse laboratory parameters in osteoporosis patients. Thus, also disclosed but not part of the invention is a diagnostic method for osteoporosis by analyzing the level of Fetuin A, respectively alpha-2-HS glycoprotein, in the respective patient.

Further features and advantages of the invention become apparent from the following description of examples in connection with the figures. The single features can be implemented individually or in combination with one another.

In the figures it is shown:
- Fig. 1: chromatogram of the inventive method using an IDA resin (IMAC);
- Fig. 2: chromatogram of the inventive method using an NTA resin (IMAC);
- Fig. 3: chromatogram of a further embodiment of the inventive method using NTA resin (IMAC);
- Fig. 4: SDS-PAGE (A) and Western Blot (B) of purified Fetuin A;
- Fig. 5: Fetuin-A levels in osteoporosis patients compared to a control group; and
- Fig. 6: Fetuin-A levels in osteoporosis patients with pathological fracture compared to osteoporosis patients without pathological fractures.

### Examples

### Example 1: Method of production of a Fetuin A preparation (alpha-2-HS glycoprotein) on IDA Resin

For purification of Fetuin A (alpha-2-HS glycoprotein) from a plasma derived preparation a BabyBio IDA column for Immobilized Metal Ion Affinity Chromatography (IMAC) (Bio-Works, Sweden) with a column volume (CV) of 5 mL has been used. The chromatography was performed at 4 °C.

### Run condition:

- Load: 36 mL (7,2 CV) feed paste IV (in 100 mM acetate buffer pH 5.0)
- DF: 39,02 mL (7,8 CV)
- Wash: 100 mL (20 CV)
- Elution: 125 mL (25 CV) gradient
- Eluat fractions: A39 - A51

### Buffers:

| | |
|---|---|
| • Column charged: | 50 mM CaCl₂ |
| • Equilibration + Wash: | 100 mM acetate buffer pH 5 |
| • Elution: | 100 mM acetate buffer + 1.4 M NaCl |
| | pH 5.0 → gradient total 25 CV |

### Result:

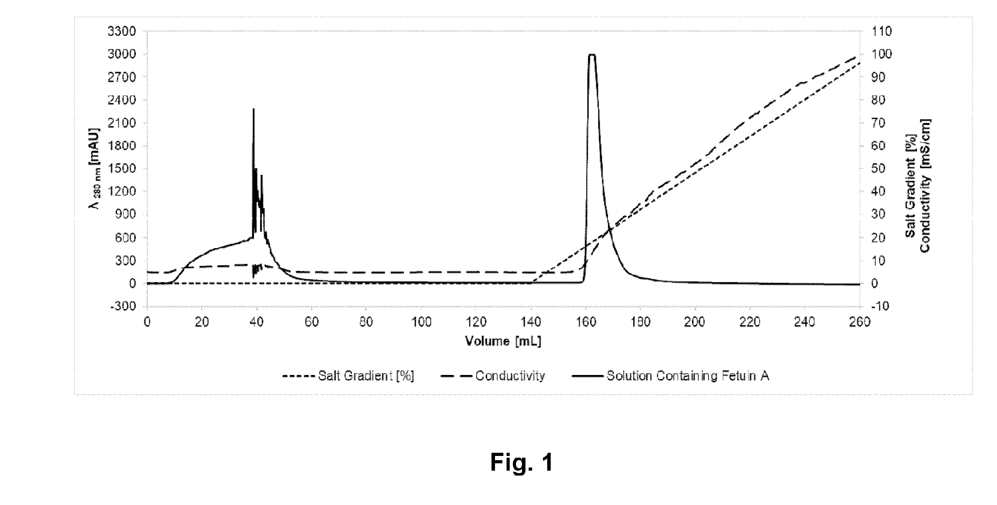

The resulting chromatogram is shown in Fig. 1. The x-axis indicates the volume in mL, the left y-axis indicates the protein content as measured by OD₂₈₀. The right y-axis indicates the conductivity in mS/cm. Fetuin A was found at a high purity in the eluted fractions A39 - A51 (corresponding to volume 159 mL - 175 mL).

### Example 2: Method of production of a Fetuin A preparation (alpha-2-HS glycoprotein) on NTA Resin

For purification of Fetuin A (alpha-2-HS glycoprotein) from a plasma derived preparation a BabyBio NTA column for Immobilized Metal Ion Affinity Chromatography (IMAC) (Bio-Works, Sweden) with a column volume (CV) of 5 mL has been used. The chromatography was performed at 4 °C.

### Run condition:

- Load: 36 mL (7,2 CV) feed paste IV (in 100 mM acetate buffer pH 5)
- DF: 39,02 mL (7,8 CV)
- Wash: 100 mL (20 CV)
- Elution: 125 mL (25 CV) gradient
- Eluat fractions: A40 - A49

### Buffers:

| | |
|---|---|
| • Column charged: | 50 mM CaCl₂ |
| • Equilibration + Wash: | 100 mM acetate buffer pH 5.0 |
| • Elution: | 100 mM acetate buffer + 1.4 M NaCl |
| | pH 5.0 → gradient total 25 CV |

### Result:

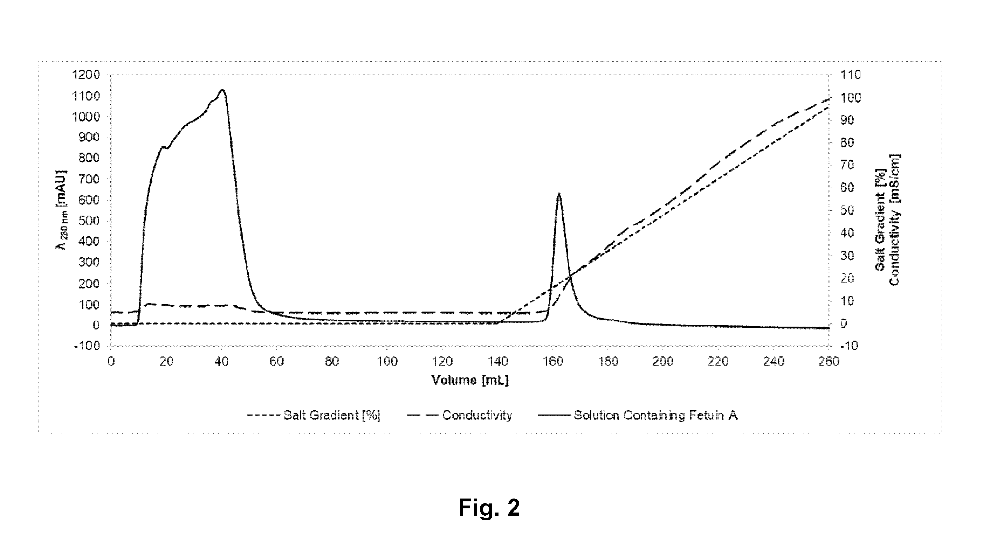

The resulting chromatogram is shown in Fig. 2. The x-axis indicates the volume in mL, the left y-axis indicates the protein content as measured by OD₂₈₀. The right y-axis indicates the conductivity in mS/cm. Fetuin A was found at a high purity in the eluted fractions A40 - A49 (corresponding to volume 158 mL - 172 mL).

### Example 3: Method of production of a Fetuin A preparation (alpha-2-HS glycoprotein) on NTA Resin

For purification of Fetuin A (alpha-2-HS glycoprotein) from a plasma derived preparation a BabyBio IDA column for Immobilized Metal Ion Affinity Chromatography (IMAC) (Bio-Works, Sweden) with a column volume (CV) of 1 mL has been used. The chromatography was performed at room temperature.

### Run condition:

- Load: 12 mL (12 CV) feed paste IV (in 10 mM Tris/HCl pH 8)
- DF: 15.02 mL (15,02 CV)
- Wash: 20 mL (20 CV)
- Elution: 15 mL (15 CV) gradient
- Eluat fractions: A7 - A12

### Buffers:

- Column charged: 50 mM CaCl₂
- Equilibration + Wash: 10 mM Tris/HCl pH 8.0
- Elution: 10 mM Tris/HCl + 0.5 M NaCl pH 8.0

### Result:

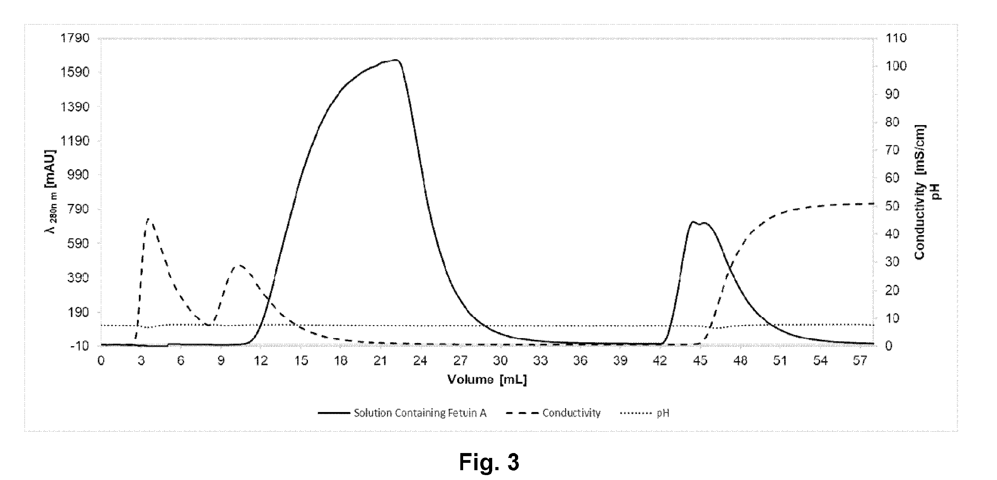

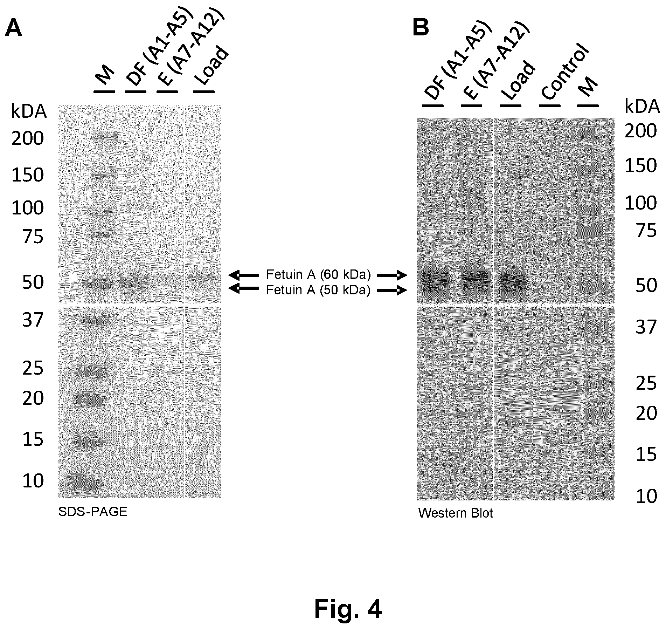

The resulting chromatogram is shown in Fig. 3. The x-axis indicates the volume in mL, the left y-axis indicates the protein content as measured by OD₂₈₀. The right y-axis indicates the conductivity in mS/cm and pH. Fetuin A was found at a purity >80% in the eluted fractions A7 - A12 (corresponding to volume 42 mL - 50 mL). The resulting SDS-PAGE (under non-reduced conditions) according to Laemmli is shown in Fig. **4A** (M - marker, DF - flow-through, E - eluate). A Western Blot using a polyclonal anti-Fetuin A antibody (BioVendor GmbH, Germany) is shown in **Fig. 4B** (M - marker, DF - flow-through, E - eluate).

### Example 4: Fetuin A levels in patients with Osteoporosis compared to a control group

### Osteoporosis group:

Patients with a proven osteoporosis (n=55), different state of illness or multiple diseases.

### Control group:

Healthy plasma donors (n=29) of both genders aged between 50 - 65 years.

### Analytical methods:

A commercial ELISA was used for the determination of Fetuin A levels. Other typical parameters for the diagnosis of osteoporosis was determined in a clinical laboratory using commercial assays.

### Results:

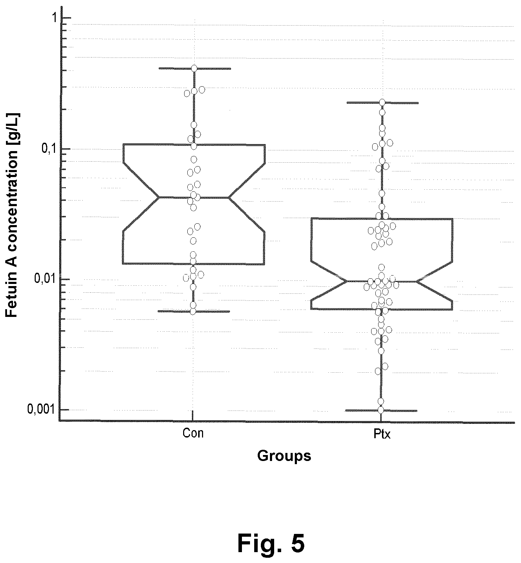

The measured Fetuin A levels of the control group (Con) and the osteoporosis group (Ptx) are shown Fig. 5. The y-axis indicates the Fetuin A concentration in blood respectively plasma in g/L. Fetuin A levels in osteoporosis patients (Ptx) showed a significant reduced level. The level of Fetuin A was reduced by a factor of 5 in comparison to the control group (Con).

The following table 1 summarize the statistical evaluation of the results. The following table 2 indicates descriptive statistics in the control and the patient group.

**Table 1: t-test for controls and samples**

| **Sample 1** | | | |
|---|---|---|---|
| Variable | Controls Fetuin A [g/L] | | |

| **Sample 2** | | | |
|---|---|---|---|
| Variable | Patients Fetuin A [g/L] | | |

| | | **Sample 1** | **Sample 2** |
|---|---|---|---|
| **Sample size** | | 29 | 55 |
| **Arithmetic mean** | | 0,08879 | 0,03316 |
| **95% CI for the mean** | | 0,04883 to 0,1288 | 0,01971 to 0,04661 |
| **Variance** | | 0,01104 | 0,002476 |
| **Standard deviation** | | 0,1051 | 0,04976 |
| **Standard** error of the **mean** | | 0,01951 | 0,006710 |
| **F-test for equal** | **variances** | | P < 0,001 |

### T-test (assuming equal variances)

| | |
|---|---|
| **Difference** | -0,05563 |
| **Pooled Standard Deviation** | 0,07348 |
| **Standard Error** | 0,01686 |
| **95% CI of difference** | -0,08918 to -0,02208 |
| **Test statistic t** | -3,299 |
| **Degrees of Freedom (DF)** | 82 |
| **Two-tailed probability** | P = 0,0014 |

**Table 2: Descriptive statistics control and patient group**

| **Variable** | **Controls Fetuin-A [g/L]** | **Patients Fetuin-A [g/L]** |
|---|---|---|
| **Sample size** | 29 | 55 |
| **Lowest value** | 0,006000 | 0,001010 |
| **Highest value** | 0,4160 | 0,2306 |
| **Arithmetic mean** | 0,08879 | 0,03316 |
| **95% CI for the Arithmetic mean** | 0,04883 to 0,1288 | 0,01971 to 0,04661 |
| **Median** | 0,04400 | 0,009933 |
| **95% CI for the median** | 0,01883 to 0,08792 | 0,008102 to 0,02196 |
| **Variance** | 0,01104 | 0,002476 |
| **Standard deviation** | 0,1051 | 0,04976 |
| **Relative standard deviation** | 1,1832 (118,32%) | 1,5006 (150,06%) |
| **Standard error of the mean** | 0,01951 | 0,006710 |
| **Coefficient of Skewness** | 1,6918 (P=0,0008) | 2,3423 (P<0,0001) |
| **Coefficient of Kurtosis** | 2,3855 (P=0,0359) | 5,3847 (P=0,0003) |

| **Percentiles** | **Controls Fetuin-A [g/L]** | **95% Confidence interval** | **Patients Fetuin-A [g/L]** | **95% Confidence interval** |
|---|---|---|---|---|
| **2,5** | 0,006000 | | 0,001157 | |
| **5** | 0,006000 | | 0,002062 | |
| **10** | 0,009400 | | 0,003413 | 0,001259 to 0,004118 |
| **25** | 0,01350 | 0,009478 to 0,03754 | 0,005766 | 0,003655 to 0,008097 |
| **75** | 0,1215 | 0,05990 to 0,2707 | 0,02980 | 0,02198 to 0,08053 |
| **90** | 0,2716 | | 0,1108 | 0,05431 to 0,1871 |
| **95** | 0,2935 | | 0,1425 | |
| **97,5** | 0,3870 | | 0,1963 | |

### Example 5: Fetuin A levels in patients with osteoporosis with and without pathological fractures

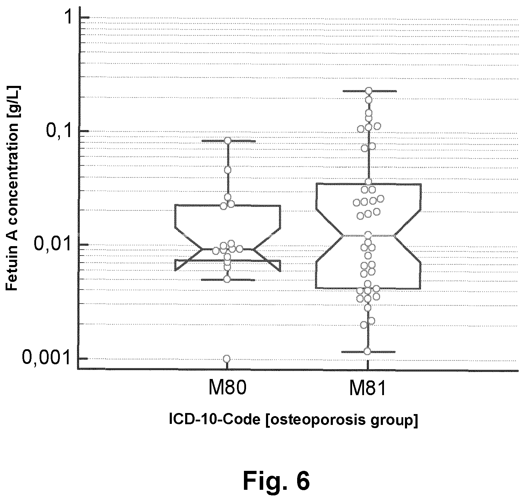

The osteoporosis group from Example 4 was divided into two subgroups, wherein according to the International Classification of Diseases (ICD) the patients were grouped into osteoporosis with pathological fracture (M80) and osteoporosis without pathological fracture (M81). The results are shown in **Fig. 6****,** wherein the y-axis indicates the Fetuin A concentration in g/L.

The statistical evaluation of the results showed that there is no significant difference (p=0.1419) in patients with a bone fracture in the medical history compared to patients without fractures.

### Example 6: Laboratory parameters in osteoporosis patients

Several blood values have been taken from the osteoporosis patients in the study. The following table 3 summarizes the results.

**Table 3: Cross correlation of osteoporosis lab parameter showing Fetuin-A as independent parameter**

| | **BAP [µg/L]** | **β-CTX [ng/mL]** | **OCal [ng/mL]** | **PTH [pg/mL]** | **VitD3 [ng/mL]** | **CRP [mg/L]** | **Fetuin-A [g/L]** | **OPon [ng/mL]** | **Calcium [mmol/L]** | **Phos [mmol/L]** | **TGF-β1 [ng/mL]** |
|---|---|---|---|---|---|---|---|---|---|---|---|
| **Age** | **0,169** | -0,310 | -0,170 | 0,149 | 0,086 | -0,030 | 0,078 | 0,169 | 0,030 | -0,106 | -0,114 |
| **BAP [µg/L]** | | 0,409 | ***0,547*** | 0,158 | -0,110 | -0,013 | 0,178 | -0,038 | 0,191 | 0,063 | -0,263 |
| **β-CTX [ng/mL]** | | | ***0,687*** | 0,224 | -0,088 | -0,053 | -0,093 | 0,045 | 0,186 | 0,076 | 0,065 |
| **OCal [ng/mL]** | | | | 0,391 | 0,061 | 0,096 | 0,077 | 0,029 | 0,101 | 0,052 | -0,083 |
| **PTH [pg/mL]** | | | | | -0,175 | -0,142 | -0,107 | 0,173 | 0,214 | -0,182 | -0,211 |
| **VitD3 [ng/mL]** | | | | | | 0,297 | -0,052 | -0,167 | -0,175 | 0,248 | 0,112 |
| **CRP [mg/L]** | | | | | | | 0,415 | 0,026 | -0,097 | -0,079 | 0,025 |
| **Fetuin-A [g/L]** | | | | | | | | 0,133 | 0,055 | -0,213 | -0,092 |
| **OPon [ng/mL]** | | | | | | | | | 0,276 | -0,246 | -0,057 |
| **Calcium [mmol/L]** | | | | | | | | | | 0,241 | -0,011 |
| **Phos [mmol/L]** | | | | | | | | | | | 0,163 |

| | | | | | | | | | | | |
|---|---|---|---|---|---|---|---|---|---|---|---|
| **BAP** = Bone- specific Alkaline Phosphatase; **β-CTX** = beta Crosslaps; **OCal** = Osteocalcin; **PTH** = Parathyroid hormone; **VitD3** = Calcidiol; **OPon** = Osteopontin; **Phos** = Phosphate; **TGF-β1** = Transforming growth factor β1 | | | | | | | | | | | |

There was no correlation to any other lab parameter used in the diagnosis of osteoporosis shown by cross-correlation. It can be concluded that Fetuin A is an independent parameter for the diagnosis of osteoporosis.

## Claims

1. Method for preparing Fetuin A from a biological liquid, comprising a chromatography step of adsorption of Fetuin A on a chelate chromatography material, wherein the chelate chromatography material is a resin which is charged with a doubly charged cation, and eluting the Fetuin A, wherein the chelate chromatography material is an immobilized metal affinity chromatography (IMAC) resin and the doubly charged cation is Ca²⁺.

2. Method of claim 1, wherein the chelate chromatography material comprises immobilized iminodiacetic acid (IDA) and/or nitrilotriacetic acid (NTA).

3. Method of claim 1 or claim 2, wherein the biological liquid is a plasma-derived preparation which is Cohn fraction IV or Cohn fraction IV after removal of albumin.

4. Method of one of the preceding claims, wherein the chelate chromatography resin is charged with CaCl₂, preferably with 10 - 100 mM CaCl₂, more preferably with 50 mM CaCl₂.

5. Method of one of the preceding claims, wherein the chromatography step comprises a washing step after loading, wherein washing is performed using a buffer not comprising any doubly charged cations.

6. Method of one of the preceding claims, wherein elution of the adsorbed Fetuin A is performed by increased ionic strength, preferably by increased NaCl concentration.

7. Method of one of the preceding claims, wherein the chromatography step is performed in the presence of at least one stabilizer for prevention of degradation of Fetuin A, wherein, preferably, the at least one stabilizer is a doubly charged cation and/or albumin.

8. Method of one of the preceding claims, wherein the purification is combined with an antiviral treatment.

9. Method of one of the preceding claims, wherein the method comprises drying or freeze-drying of the eluted Fetuin A.

10. Pharmaceutical composition comprising a protein preparation obtainable by a process according to one of the claims 1 to 9 for use in treatment or prophylaxis in patients suffering from Fetuin A deficiency, wherein the preparation comprises at least 60% (w/w) human Fetuin A being sourced from human adult plasma and at least one pharmaceutically acceptable carrier and wherein the process for preparation is combined with one or more antiviral treatments.

11. Pharmaceutical composition for use according to claim 10, wherein the preparation comprises at least 80% (w/w) human Fetuin A.

12. Pharmaceutical composition for use according to claim 10 or claim 11 for use in treatment or prophylaxis in patients suffering from osteoporosis and/or in patients on risk of bone fractures and/or in patients on risk of hypercalcification and/or in patients on risk of endochondral extraosseous or heterotopic ossification.

## Patentansprüche

1. Verfahren zur Herstellung von Fetuin A aus einer biologischen Flüssigkeit, umfassend einen Chromatographieschritt mit einer Adsorption von Fetuin A an einem Chelatchromatographiematerial, wobei das Chelatchromatographiematerial ein Harz ist, das mit einem doppelt geladenen Kation beladen ist, und Elution des Fetuin A, wobei das Chelatchromatographiematerial ein Harz für eine immobilisierte Metallaffinitätschromatographie (IMAC) ist und das doppelt geladene Kation Ca²⁺ ist.

2. Verfahren nach Anspruch 1, wobei das Chelatchromatographiematerial immobilisierte Iminodiessigsäure (IDA) und/oder Nitrilotriessigsäure (NTA) umfasst.

3. Verfahren nach Anspruch 1 oder Anspruch 2, wobei die biologische Flüssigkeit ein aus Plasma gewonnenes Präparat ist, das Cohn-Fraktion IV oder Cohn-Fraktion IV nach Entfernung von Albumin ist.

4. Verfahren nach einem der vorhergehenden Ansprüche, wobei das Chelatchromatographieharz mit CaCl₂ beladen ist, vorzugsweise mit 10 - 100 mM CaCl₂, noch bevorzugter mit 50 mM CaCl₂.

5. Verfahren nach einem der vorhergehenden Ansprüche, wobei der Chromatographieschritt einen Waschschritt nach dem Beladen umfasst, wobei das Waschen unter Verwendung eines Puffers durchgeführt wird, der keine doppelt geladenen Kationen umfasst.

6. Verfahren nach einem der vorhergehenden Ansprüche, wobei die Elution des adsorbierten Fetuin A durch erhöhte Ionenstärke, vorzugsweise durch erhöhte NaCl-Konzentration, durchgeführt wird.

7. Verfahren nach einem der vorhergehenden Ansprüche, wobei der Chromatographieschritt in Gegenwart mindestens eines Stabilisators zur Verhinderung eines Abbaus von Fetuin A durchgeführt wird, wobei der mindestens eine Stabilisator vorzugsweise ein doppelt geladenes Kation und/oder Albumin ist.

8. Verfahren nach einem der vorhergehenden Ansprüche, wobei die Reinigung mit einer antiviralen Behandlung kombiniert wird.

9. Verfahren nach einem der vorhergehenden Ansprüche, wobei das Verfahren ein Trocknen oder ein Gefriertrocknen des eluierten Fetuin A umfasst.

10. Pharmazeutische Zusammensetzung, umfassend ein Proteinpräparat, das durch ein Verfahren gemäß einem der Ansprüche 1 bis 9 erhältlich ist, zur Verwendung bei der Behandlung oder Prophylaxe bei Patienten, die an einem Fetuin-A-Mangel leiden, wobei das Präparat mindestens 60 % (w/w) humanes Fetuin A, das aus humanem adulten Plasma stammt, und mindestens einen pharmazeutisch akzeptablen Träger umfasst und wobei das Verfahren zur Herstellung mit einer oder mehreren antiviralen Behandlungen kombiniert wird.

11. Pharmazeutische Zusammensetzung zur Verwendung gemäß Anspruch 10, wobei das Präparat mindestens 80 % ( w/w) humanes Fetuin A umfasst.

12. Pharmazeutische Zusammensetzung zur Verwendung gemäß Anspruch 10 oder Anspruch 11 zur Verwendung bei der Behandlung oder Prophylaxe bei Patienten, die an Osteoporose leiden, und/oder bei Patienten mit einem Risiko für Knochenbrüche und/oder bei Patienten mit einem Risiko für Hyperkalzifizierung und/oder bei Patienten mit einem Risiko für endochondrale extraossäre oder heterotope Ossifikation.

## Revendications

1. Procédé de préparation de Fétuine A à partir d'un liquide biologique, comprenant une étape de chromatographie d'adsorption de Fétuine A sur un matériel chromatographique de type chélate, dans lequel le matériel chromatographique de type chélate est une résine chargée avec un cation doublement chargé, et l'élution de la Fétuine A, dans lequel le matériel chromatographique de type chélate est une résine de chromatographie d'affinité sur métaux immobilisés (IMAC) et le cation doublement chargé est Ca²⁺.

2. Procédé selon la revendication 1, dans lequel le matériel chromatographique de type chélate comprend de l'acide iminodiacétique (IDA) et/ou de l'acide nitrilotriacétique (NTA) immobilisés.

3. Procédé selon la revendication 1 ou la revendication 2, dans lequel le liquide biologique est une préparation dérivée de plasma qui est une fraction de Cohn IV ou une fraction de Cohn IV après élimination de l'albumine.

4. Procédé selon l'une des revendications précédentes, dans lequel la résine chromatographique de type chélate est chargée avec CaCl₂, de préférence avec 10 à 100 mM de CaCl₂, plus préférentiellement avec 50 mM de CaCl₂.

5. Procédé selon l'une des revendications précédentes, dans lequel l'étape de chromatographie comprend une étape de lavage après le chargement, dans lequel le lavage est réalisé en utilisant un tampon ne comprenant aucun cation doublement chargé.

6. Procédé selon l'une des revendications précédentes, dans lequel l'élution de la Fétuine A adsorbée est réalisée par force ionique augmentée, de préférence par concentration en NaCl augmentée.

7. Procédé selon l'une des revendications précédentes, dans lequel l'étape de chromatographie est réalisée en présence d'au moins un stabilisant pour prévenir une dégradation de la Fétuine A, dans lequel, de préférence, l'au moins un stabilisant est un cation doublement chargé et/ou l'albumine.

8. Procédé selon l'une des revendications précédentes, dans lequel la purification est associée à un traitement antiviral.

9. Procédé selon l'une des revendications précédentes, dans lequel le procédé comprend un séchage ou une lyophilisation de la Fétuine A éluée.

10. Composition pharmaceutique comprenant une préparation de protéines pouvant être obtenue par un procédé selon l'une des revendications 1 à 9 pour une utilisation dans le traitement ou la prophylaxie chez des patients souffrant d'un déficit en Fétuine A, dans lequel la préparation comprend au moins 60 % (p/p) de Fétuine A humaine dérivée de plasma humain adulte et au moins un véhicule pharmaceutiquement acceptable et dans laquel le procédé de préparation est combiné à un ou plusieurs traitements antiviraux.

11. Composition pharmaceutique pour une utilisation selon la revendication 10, dans laquelle la préparation comprend au moins 80 % (p/p) de Fétuine A humaine.

12. Composition pharmaceutique pour une utilisation selon la revendication 10 ou la revendication 11 pour une utilisation dans le traitement ou la prophylaxie chez des patients souffrant d'ostéoporose et/ou chez des patients présentant un risque de fractures osseuses et/ou chez des patients présentant un risque d'hypercalcification et/ou chez des patients présentant un risque d'ossification endochondrale, extra-osseuse ou hétérotopique.
